# EUROPEAN PATENT APPLICATION

(11) **EP 3 118 724 A1**
(43) Date of publication of application: **18.01.2017**
(21) Application number: 15761852.1
(22) Date of filing: 10.03.2015
(51) Int. Cl.: G06F 3/01, G06F 1/16

(54) **TEXTILE MOTHERBOARD, HAVING A MODULAR AND INTERCHANGEABLE DESIGN, FOR MONITORING, REPORTING AND CONTROLLING**

(30) Priority: 10.03.2014 MX 2014002826
(71) Applicant: Vacas Jacques, Paulino, 37150 León, Guanajuato (MX)
(72) Inventor: Vacas Jacques, Paulino, 37150 León, Guanajuato (MX)
(74) Representative: Pons Ariño, Angel
(86) International application number: PCT/MX2015/000040
(87) International publication number: WO 2015/137795

(57) **Abstract**

The invention relates to a textile motherboard (TMT) that can be used in, inter alia, clothing, coats, and dressings gowns, and which includes at least one central processing unit (CPU) or a peripheral or a combination of both, for monitoring, reporting and controlling parameters of the wearer. The clothing can be used or worn by a human or non-human user. The textile of the clothing serves as a substrate for forming the TMT. The TMT can have multi-layer structures and VIAs (vertical interconnect access). The traces of the TMT are formed of textile material that can transmit signals between the CPU and information storage means, or the combination with the peripheral(s). The layers, traces and VIAs are incorporated using known textile-handling techniques such as weaving, stamping, or same can be printed onto the textiles. Each component is modular and interchangeable, and is connected to the TMT using textile connectors that can be clips, hooks or similar elements. The TMT, the CPU and the peripherals are washable. The CPU and each peripheral can be mounted on rigid or flexible textile boards or PCBs (printed circuit board), using discrete electronic or photonic elements. The CPU includes a micro-controller, a micro-processor or a comparable element. The peripherals include photonic transducers or electronic transducers, or a combination of both, such as, inter alia, capacitive sensors, pulse meters, humidity sensors, thermometers, accelerometers, gyroscopes. The peripherals also include: screens; modules for serial communication via radio frequency (including, inter alia, Bluetooth, Zigbee technology), Wi-Fi; and similar elements

## Description

### FIELD OF THE DISCLOSURE.

The present disclosure relates to exemplary embodiments of a textile motherboard arrangement, and more particularly to textile motherboards that may be employed in garments, blankets, towels, tablecloths, gowns, etc., with non exclusive applicability to the medical, commercial, family-care, or sports fields.

### MAIN OBJECTIVE OF THE INVENTION.

A main object of the present invention is to render intelligent textiles that may be worn or utilized by users with the end goal of monitoring, informing, or controlling parameters of interest.

The textiles are rendered intelligent by incorporating into them at least one textile motherboard TMB, which can function with analog- and/or digital- electric and/or photonic signals. Furthermore, at least one central processing unit CPU or at least one peripheral may be connected to the TMB, in order to provide one of the abovementioned features to the intelligent textile. In order to bestow flexibility, the TMB design is modular; furthermore, both CPU and peripherals may be utilized interchangeably.

In a first exemplary arrangement of the TMB including CPU and peripherals, the intelligent textile may be able to perform monitoring. For instance, an intelligent textile with a TMB may be capable of monitoring user parameters such as temperature, the selection from a menu of options, solar exposure, pulse, etc.

In another exemplary embodiment of the TMB including CPU and peripherals, the intelligent textile may enable the person wearing it to inform her about events. For instance, an intelligent textile with a TMB may be able to inform the person wearing it about the presence of humidity by means of visual, vibrating, and audible alarms. Furthermore, an intelligent textile with a TMB may inform a portable device, such as a tablet or a smartphone, about further events of the person wearing it, for example during sports events.

Finally, in yet another exemplary arrangement of the TMB including CPU and peripherals designed to control, the intelligent textile may serve as a platform to manipulate various objects of interest. For example, an intelligent textile with a TMB may be able to control the call for assistance to a third person, after e.g. selecting a menu of options; additionally, a comparable embodiment may enable to control the lights of a public establishment. Furthermore with an adequate configuration of peripherals in a TMB, the operation of determined devices (like tablets or similar equipment) may be controlled, achieving the interaction between a user and the aforementioned devices, by means for instance of an application.

### BACKGROUND INFORMATION.

There exist garments with three electrodes to monitor physiological parameters, such as the arrangement disclosed in the document US 2007/0078324 A1, and granted to Ravindra Wijisiriwardana, which consists of a system or a garment that comprises at least three electrodes to monitor at least one physiological event of the person wearing it. Specially, one electrode is utilized to send an inverted noise signal as a feedback mechanism to eliminate the noise generated in the detection process. This system is particularly designed to measure the electrical characteristics of the user, such as cardiograms or cardiac frequency.

Another example of a comparable device is depicted in the document US 8,340,740 B2 granted to Christian Holzer et. al., which consists of a garment that enables physiological monitoring. The measuring sensors are integrated into the garment. The device that monitors the physiological properties is located on the back of the garment, and may be integrated and fixed to the garment. It may also be detached from the garment.

A further instance of this type of devices is described in document US 2003/0212319 A1 granted to Alan Remy Magill, which consists of a physiological monitoring garment, wherein the electricity is conducted by means of fibers from the skin surface to a garment that has a microprocessor, telemetry system, and power supply to monitor and transmit electrocardiogram data. The garment with the microprocessor may be detached, thus enabling the cleaning of the garment in contact with the skin. The system may also be used in reverse order, in order to provide electrical stimulation to the body.

Another example of these types of devices is shown in the document US 2012/0136231 A1 granted to Gal Markel, which consists of a garment that provides physiological and environmental monitoring, as well as location information, and discloses a garment or system of garments with the capacity to monitor health. The garment comprises a variety of electrocardiogram sensors, other sensors to monitor health, a processor, conductive fibers, as well as a communication unit in order to send physiological, environmental, and location data.

A final instance of this kind of devices is described in the document WO 2011/131235 A1 granted to Javier Guillen Arredondo et. al., which consists of a monitoring system. This proposal is composed of a monitoring system with one or more sensors adapted to measure one or more parameters, indicative of the physical health of the user. The proposal also includes a system to collect the data and an evaluation system to compare the values with predetermined information. At least one of the sensors is incorporated into a garment.

### TECHNICAL PROBLEM TO SOLVE.

Despite the fact that garments exist to monitor physiological parameters, the approaches known in the state-of-the-art do not have a textile motherboard scheme incorporated into the design of the devices.

Moreover even though some devices are detachable, they are not modular and none describe the feature of being interchangeable. For instance, once the device is designed to monitor cardiac frequency, that same design is not able to monitor another parameter, such as temperature, therefore requiring the use of a completely new device.

Also absent in the state-of-the-art is the ability of the textiles to manipulate objects of interest, such as the lights of a public establishment or the interaction with a device or computer application, after selecting a, preferably, textile menu of options.

### BRIEF DESCRIPTION OF THE INVENTION.

In order to address the abovementioned problem in the state-of-the-art, it is the object of the present invention to disclose embodiments that provide the structure of a textile motherboard (TMB) The substrate of the TMB, object of the present invention, is conformed of textile material. Furthermore, the TMB may be structured with single-layer or multiple-layer designs, incorporating perforations for layer interconnection. Layers and perforations are conformed of textile materials and are incorporated into the TMB by means of textile manipulation techniques.

The routing of the TMB includes at least two different types of routing: maze routing and X-Y routing. Both routing types may be implemented in the TMB, for instance by utilizing textile printing techniques or by incorporating the routes by following a required pattern using textile manipulation techniques.

The peripherals may be broadly classified as input or output peripherals. In order to yield flexibility, the TMB incorporates terminals in the CPU and peripherals that simplify the modular and interchangeable design of the TMB by employing conventional textile connectors.

Finally, the implementation of an interchangeable TMB design, including input and output peripherals, enables the user to manipulate objects of interest, such as the lights of a public establishment, after selecting a menu of options. Similarly, the user is enabled to interact with devices that me be controlled by means of a determined arrangement of peripherals adequately employed in the TMB, which may be conformed as a garment or other textile.

The TMB consists of at least one layer of conductive textile material that conforms a textile peripheral, which may transmit at least one signal to a controller placed in a detachable and interchangeable fashion within the TMB.

The peripherals may exhibit different configurations depending on the desired action to perform; exemplary embodiments include battery holders, buses (also referred to as routings), connectors, jumpers, pads, sockets, switches, VIAs, etc.

Exemplary textile peripherals include normally-open and normally-closed switches, exhibiting at least one pole and one throw (1P1T), one pole and two throws (1P2T), one pole and three throws (1P3T), which are conformed of conductive layers, made out of pads or contact regions, and an isolating layer placed between the conductive layers or pads. The isolating layer is configured to generate specific contact areas and to avoid accidental contacts between the conductive textiles. The design of the present invention endows the switch with regions of null conductivity and regions of maximum conductivity within the peripheral.

Exemplary peripherals employable in the conformation of intelligent textiles may be achieved by using elements conventionally utilized in the textile industry, such as snaps, fasteners, hooks, pins, etc. A specific embodiment of an *N*-pole and an *N*-throw (NPNT) switch may be configured by utilizing commonly available textile elements, such as a conductive zip or slide fastener, wherein each metallic or conductive tooth of the zip or slide portion may be employed as a pole or a throw. In another exemplary embodiment, the pins could perform the function of contact nodes for peripherals of a determined TMB.

Other exemplary peripherals such as buses are configured as contact or connection ports for different elements, which may employ snap or hook elements for the interconnection, both commonly utilized in the textile industry. Further exemplary peripherals such as pads may be utilized to ensure adequate contact between components, such as snaps and buses.

Depending on the desired configuration and the number of ports or buses to use, a determined amount of snaps or hooks, as well as pads, are utilized. These components are put into place and adhered to the base (isolating) or conducting textiles by means of conventional textile manipulation techniques, such as manual or mechanical sewing, using conductive textiles to achieve the required connectivity.

Additional exemplary textile peripherals may be conformed as sockets by utilizing conductive routings or printings over a textile substrate, with perforations or connectors to accommodate the desired photonic integrated circuits (PICs), electronics integrated circuits, or additional elements. This type of peripheral incorporates snaps and is therefore replaceable and modular, thus enabling the TMB to accommodate other peripherals that exhibit different functionalities by simply interchanging this socket, or the module that it contains.

According to an embodiment of the present disclosure, exemplary combinations of different switches may be employed to control an electric or electronic device, such that a user may interact with the device. In this manner, the invention in a preferred embodiment may be configured as a garment, preferably a shirt, which enables a user to interact with and control a device, and that may be used as an infant learning stimulation system.

Another embodiment of the present disclosure may be configured as a bedroom textile or a bedroom linen, such as a bedcover or a bed mattress, in order to monitor the presence of elderly people, and plausibly including humidity detectors.

Other embodiments of the present disclosure are configured as bracelets that enable the measurement of temperature, as well as other parameters of interest, from a user.

Another embodiment of the present disclosure is configured as a garment for infants, which is adapted to monitor and measure vital signs, humidity, movement, in addition to other infant parameters.

The aforementioned devices may additionally accommodate a radio frequency communications module (employing Zigbee, Bluetooth, etc.), or a "Wi-Fi" communications module, and similar, designed with textile components, and intended to be used interchangeably within the TMB.

### BRIEF DESCRIPTION OF DRAWINGS.

Figure 1A.- Shows a design of a plausible embodiment of a single-layer textile motherboard (TMB);
Figure 1 B.- Depicts the cross section of a plausible embodiment of a multiple-layer textile motherboard wherein the use of "VIAs" for the interconnection between the multiple layers is highlighted;
Figure 2A.- Illustrates the utilization of textile materials to conform a TMB, such as the embodiment depicted in Figure 1A;
Figure 2B.- Shows the cross section of an embodiment of a multiple-layer textile motherboard wherein the layers are incorporated into the front- and back- parts of the textile, and are isolated in the middle part;
Figure 2C.- Depicts the implementation of X-Y routing in a textile garment, assuming that the routing is performed on the top layer of the motherboard;
Figure 3.- Shows a layout embodiment of an intelligent vest with exchangeable input and output devices;
Figure 4.- Illustrates the employment of conventional textile connectors that facilitate the modular and exchangeable design;
Figure 5.- Depicts an embodiment in the form of an intelligent tablecloth, which enables the user to manipulate objects of interest, such as the lights of a public establishment, after selecting a (textile) menu of options;
Figure 6.- Illustrates a 1-pole and 1-throw textile switch, which may function as a peripheral of the TMB;
Figure 6A.- Depicts an isolating layer with uniform pattern for the exemplary textile switch shown in Figure 6, the switch exhibits a uniform active area;
Figure 7.- Illustrates a 1-pole and 2-throws textile switch, which may function as a peripheral of the TMB;
Figure 7A.- Shows an isolating layer with a divided pattern for the exemplary textile switch depicted in Figure 7, the switch exhibits two individual active areas;
Figure 8.- Illustrates a 1-pole and 3-throws textile switch, which may function as a peripheral of the TMB;
Figure 8A.- Depicts an isolating layer with three sections for the exemplary textile switch shown in Figure 8, the switch exhibits three active sections;
Figure 9.- Shows the design of a TMB for a learning stimulation garment for infants, the TMB incorporates textile logic gates to enable combinational logic;
Figure 10.- Illustrates the implementation of a communications module socket based on the TMB;
Figure 11A.- Depicts a connector socket or a socket to connect peripherals within a TMB, the shown connectors are snaps, known in the art of textile manipulation;
Figure 11B.- Shows a connector socket or a socket to connect peripherals within a TMB, the depicted connectors are hooks, known in the art of textile manipulation;
Figure 11C.- Depicts a connector socket or a socket to connect peripherals within a TMB, the shown connectors are hook and loop systems, such as Velcro®, known in the art of textile manipulation;
Figure 12.- Depicts the implementation of contact zones or pads conformed of textile material, the pads are employed to join components, such as snaps or pins, with the buses or routings of the TMB;
Figure 13.- Illustrates the TMB design of a temperature monitoring bracelet, the TMB includes a 2-poles and 2-throws switch implemented with hook and loop connectors, such as Velcro®;
Figure 14.- Depicts the design of a TMB for a battery holder, which may function as an individual or embedded TMB peripheral;
Figure 15.- Illustrates the TMB design for the monitoring of bedroom textiles or linens, such as a bedcover or a bed sheet or a bed mattress, the TMB may incorporate textile logic gates to enable combinational logic for the detection of fluids or presence;
Figure 16.- Shows the design of a TMB for an infant garment that may monitor humidity or temperature or movement.

### DETAILED DESCRIPTION OF THE INVENTION.

The present disclosure relates to an apparatus that may be employed in garments, blankets, towels, tablecloths, gowns, etc., which may consist of a *first element* (100), a *second element* (200), a *third element* (300), a *fourth element* (400) and a *fifth element* (500).

The *first element* (100) consists of a textile motherboard (100), TMB. The design of the TMB is modular and permits to interchange elements within the TMB. The TMB utilizes connectors to incorporate exemplary components such as central processing units (102) or peripherals (103).

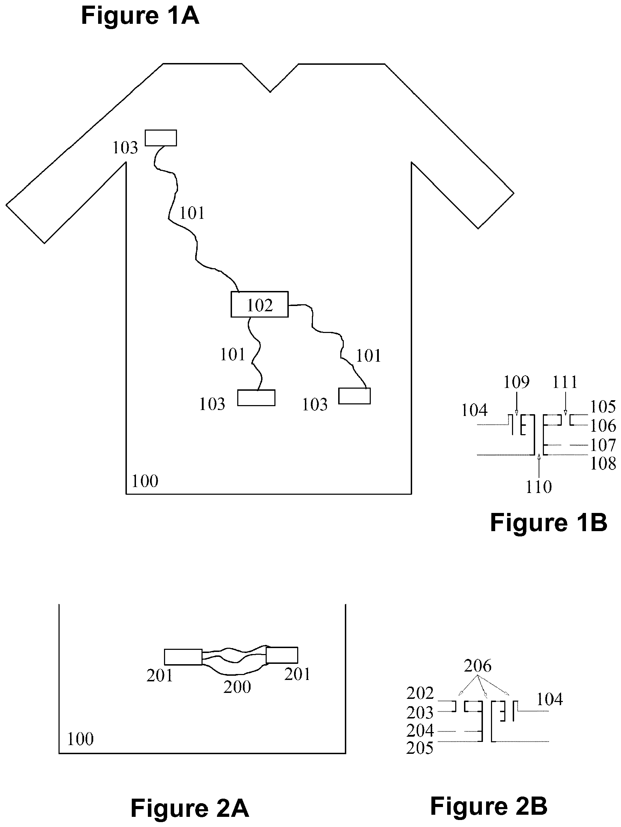

An exemplary embodiment of the present disclosure of the TMB (100) is depicted in Figure 1A. According to this exemplary embodiment, the TMB (100) consists of at least one substrate layer, with at least one conductive routing (101), and at least one terminal connected to the at least one conductive routing wherein exemplary components, such as a CPU (102) or a peripheral (103) or a combination thereof, may be installed.

The TMB (100) defines an electronic or a photonic circuit, which is a function of the arrangement of peripherals (103) and CPU (102) utilized, wherewith information may be monitored, manipulated, as well as emitted with the object of signalizing, or informing, or controlling depending on the intended purpose of the product that incorporates the TMB.

In Figure 1B, an exemplary embodiment of the TMB is shown to exhibit a multiple-layer structure (104) in addition to perforations for the interconnection between layers (109, 110, 111); known as Vertical Interconnect Accesses or "VIAs" to those skilled in the art.

A first exemplary layer (105) may serve as a platform to guide a first digital or analog signal. A second exemplary layer (106) may serve as a platform to guide a second digital or analog signal. A third exemplary layer (107) may serve as a platform to provide a constant electric or photonic signal. A fourth exemplary layer (108) may serve as a platform to provide a reference.

The foregoing descriptions of the third and fourth exemplary layers could for instance provide +5V and 0V signals, respectively. The aforementioned layers may be employed repetitively, as deemed necessary by a design. This layer structure is illustrative and does not limit the embodiments of a particular TMB.

According to the exemplary embodiment of the present disclosure shown in Figure 1 B, layer interconnection ensues by utilizing perforations (109, 110, 111) or Vertical Interconnect Accesses, known as "VIAs" to those skilled in the art. A first exemplary VIA that can be employed is the tag VIA (109). A second exemplary VIA that may be utilized is the thru VIA (110). A third exemplary VIA that can be used is the sequential VIA (111). Additional exemplary VIAs that may be employed include photo-defined, controlled depth, or buried VIAs. This list of VIAs is illustrative and does not limit the embodiments of a particular TMB.

The *second element* (200) consists of textile material with the capacity to transmit and isolate digital- and/or analog- electric and/or photonic signals, or a combination thereof. In Figure 2A an exemplary embodiment of a TMB substrate, which is conformed of textile material, is depicted. In such exemplary substrate (100), routing structures (200) may be incorporated to guide signals to and from components (201)

The routing structures (200) within the TMB (100) may consist of electric textile conductors, a textile arrangement that incorporates fiber optics or waveguides, as well as printed or stamped textiles with the conductive routing.

An exemplary TMB may consist of knitted or weaved textiles, such as those known in the art, which define a substrate layer (100), and wherein conductive textiles (200) may be intercalated appropriately, in order to define the intended routings for the circuit. Exemplary conductive textiles (200) may extend from a terminal up to a CPU (201) or may exhibit a desired extension for a specific function, thus defining a determined conductive routing.

According to the exemplary embodiment of the present disclosure shown in Figure 2B, a TMB may implement multiple textile structures, such as knitted or weaved textiles, as well as stampings or printings that serve as layers (104), and that define independent conductive routings (202, 203, 204, 205), wherein isolating layers may be superposed in order to avoid interference between routings, and wherein the interconnection between the different conductive routings may be done by means of textile VIAs (206)

According to one exemplary embodiment of the present disclosure, a TMB may incorporate a substrate layer (100) that simultaneously functions as an isolating layer. Moreover, the configuration of knitted or weaved textiles, stampings, or printings (200), which define the conductive routings, may be placed adjacent one to the other in a single plane, separated by isolating layers. According to still another exemplary embodiment of the TMB, a configuration of knitted or weaved textiles, stampings, or printings (104), which define the conductive routings, may be placed superposed (202, 203, 204, 205) in an alternating manner with isolating layers.

Exemplary interconnections between knitted or weaved textiles localized in a single layer can be implemented by employing other knitted or weaved textiles, which define the aforementioned exemplary interconnections. Exemplary interconnections between printings localized in a single layer can be implemented by employing other printings, which define the aforementioned exemplary interconnections. Exemplary interconnections between stampings localized in a single layer can be implemented by employing other stampings, which define the aforementioned exemplary interconnections.

Exemplary interconnections between knitted or weaved textiles localized in multiple layers can be implemented by employing other knitted or weaved textiles, which define the aforementioned exemplary interconnections. Exemplary interconnections between printings localized in multiple layers can be implemented by employing other printings, which define the aforementioned exemplary interconnections. Exemplary interconnections between stampings localized in multiple layers can be implemented by employing other stampings, which define the aforementioned exemplary interconnections.

The exemplary conductive routings (200), layers (202, 203, 204, 205), and VIAs (206) are conformed of textile material and are implemented by employing textile manipulation techniques. A first layer (202) may be incorporated into the front section of a textile. A second layer (203) and a third layer (204) may be isolated. A fourth layer (205) may be incorporated into the back section of a textile. Moreover, each of the first (202), second (203), third (204), and fourth (205) layers may be conformed by utilizing individual textiles.

The exemplary conductive routings (200), layers (202, 203, 204, 205), and VIAs (206) of a TMB (100) may be implemented by employing known textile manipulation techniques such as knitting, weaving, stamping, perforating, or may also be printed on the textiles.

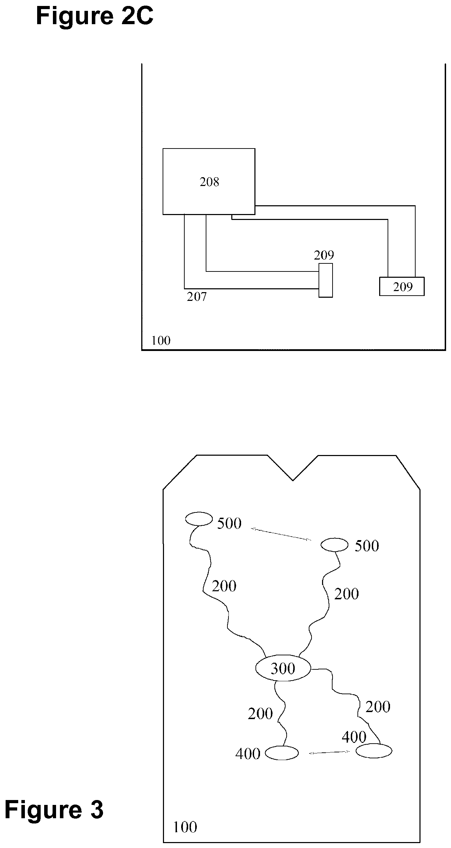

The exemplary embodiment of the present disclosure shown in Figure 2C depicts the routing of a TMB (100). The routing of a TMB (100) includes at least two different types of routings: maze routing and X-Y routing (207). Both routing types may be implemented in a TMB (100), for instance by utilizing textile printing techniques or by incorporating the routes by following a required pattern using textile manipulation techniques. The routing guides the signals between the CPU (208) and a means to register information (209) or between the CPU and the combination of peripherals (209).

The *third element* (300), *fourth element* (400), and *fifth element* (500) consist of a CPU (300), input peripherals (400), and output peripherals (500) connected to a TMB (100), which are shown in an exemplary embodiment in Figure 3. Such exemplary embodiment of a TMB (100) may be characterized by allowing the interchange of CPU (300) or peripheral elements (400, 500), in order to monitor different variables, signalize, inform, or control. In this exemplary embodiment, TMB (100), routing (200), CPU (300), and peripherals (400, 500) are washable. Exemplary CPU (300) and peripherals (400, 500) may be mounted on textile boards, as well as rigid or flexible printed circuit boards PCBs. Each exemplary board may incorporate discrete electronic elements (such as resistors, integrated circuits, capacitors, etc.) or discrete photonic elements (such as Bragg gratings, beam dividers, interferometers, etc.)

An exemplary CPU (300) may consist of a photonic or an electronic device that processes signals sent by the peripheral elements, and sends information by employing appropriate peripheral elements. Exemplary CPUs (300) may include a microcontroller or a microprocessor or a comparable element.

The CPU (300) connects with the peripherals (400, 500) employing the textile routing (200). The TMB (100) peripherals (400, 500) may be broadly classified as input (400) and output (500) devices.

Exemplary input peripherals (400) consist of elements such as photonic transducers or electronic transducers or combinations thereof. Therefore, exemplary input peripherals (400) may include capacitive sensors or temperature sensors or accelerometers or respiratory frequency sensors or humidity sensors or magnetometers or chest expansion sensors or gyroscopes or pulse sensors or muscular activity sensors or similar devices.

Exemplary output peripherals (500) may include elements like textile transducers or a screen or a vibration device or an audible device or an illuminating device or a device capable of emitting information or a memory module or a serial communications module or a radio frequency communications module (using Zigbee technology, Bluetooth, etc.) or a "Wi-Fi" communications module or similar devices.

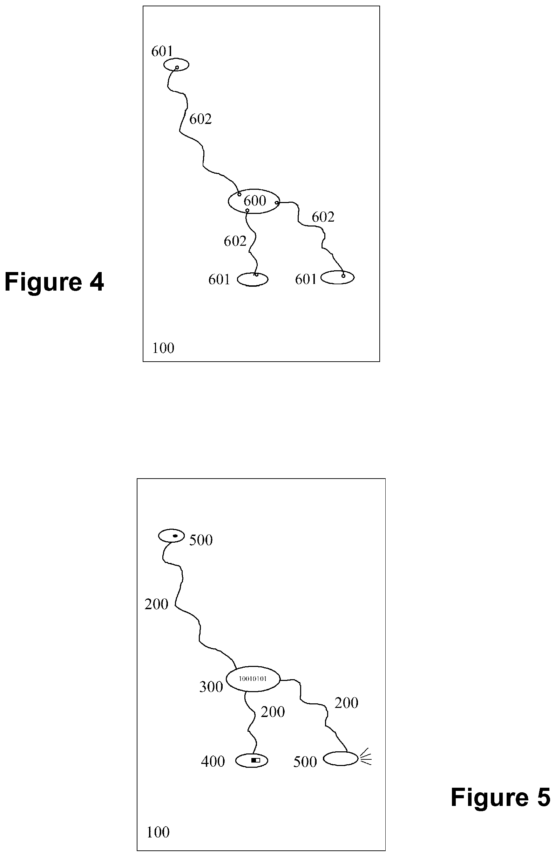

In order to yield flexibility, the TMB (100) incorporates terminals, in the CPU (600) and peripherals (601), which simplify the modular and interchangeable design by employing conventional textile connectors such as snaps, hooks and eyes, hooks and loops (Velcro®), or similar elements. This aspect of the invention is illustrated in the exemplary embodiment of Figure 4.

Furthermore, the exemplary terminals exhibit appropriate characteristics in order to be able to interchange the CPU (600) or the peripheral elements (601) to monitor different variables, signalize, inform, or control.

Each terminal is adhered to a determined routing, by using textile manipulation techniques, which enables the conduction of signals between elements of a TMB. For instance, an exemplary terminal in the form of a snap (601) may be sewed to the corresponding routing (602) to facilitate the communication between a peripheral (601) and a CPU (600).

A final exemplary embodiment of the present disclosure, shown in Figure 5, depicts a TMB (100), including routing (200), CPU (300), input (400), and output (500) peripherals to enable a user to manipulate objects of interest, such as the lights of a public establishment, after selecting a (textile) menu of options.

The foregoing merely illustrates the principles of the disclosure. Various modifications and alterations to the described embodiments will be apparent to those skilled in the art in view of the teachings herein.

### PREFERRED APPROACHES TO IMPLEMENT THE INVENTION.

By utilizing a textile motherboard (TMB) conformed of specific CPU and peripherals, a garment may be able to monitor, inform, and control. For instance, an intelligent textile in the form of a gown with a peripheral, periodically scanned by a CPU, may be utilized to monitor temperature and inform about the presence/absence of fever, and may control with a button the call for assistance. Moreover, due to the modular and interchangeable design, the input peripheral may, for instance, be changed from a temperature detector to a pulse detector.

By employing a determined arrangement of TMB, CPU, and peripherals, an intelligent textile may enable the person wearing it to inform her about events. For instance, an intelligent textile may be able to inform about the presence of humidity by means of visual, vibrating, and audible alarms. Furthermore, an intelligent textile may be capable of informing a portable device, such as a tablet or a smartphone, about further events of the person wearing it, for example during sports events.

Finally, by implementing a specific arrangement of TMB, CPU, and peripherals designed to control, an intelligent textile may serve as a platform to manipulate various objects of interest. For example, an intelligent textile may be able to control the call for assistance to a third person, after selecting a menu of options; as well as enabling the control of lights of a public establishment.

The TMB is designed on textile substrates; the textile substrate may function as an isolating substrate or as a support to incorporate the required peripherals, as well as additional components.

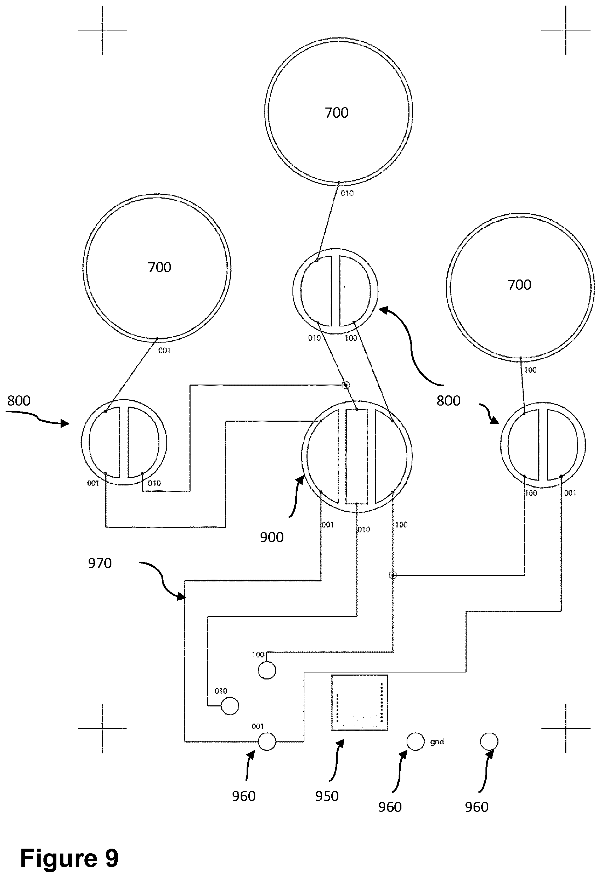

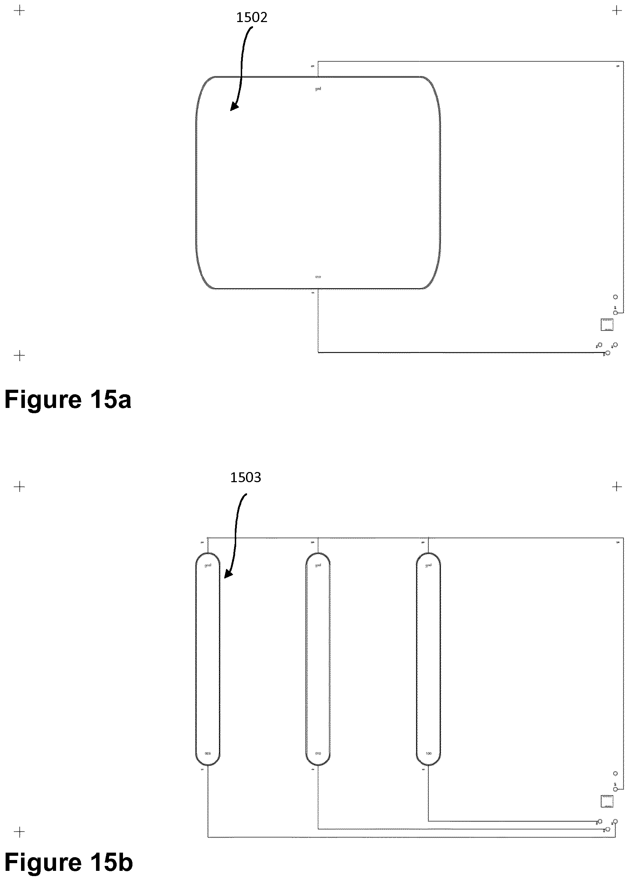

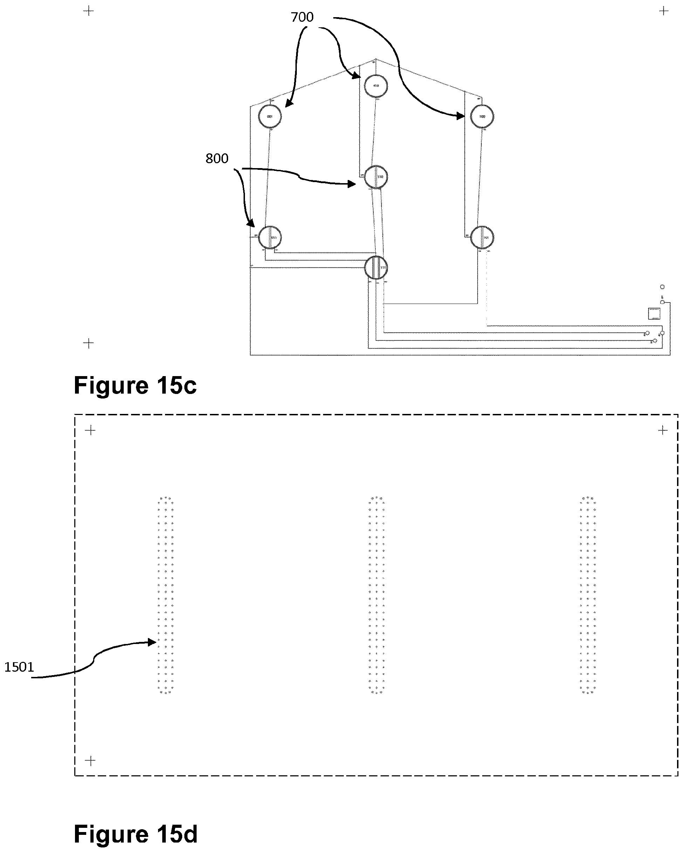

The utilization of combinational logic enables this kind of textile technology to be configured in order to enact specific functions, for instance, in an exemplary embodiment 1-, 2-, or 3-throw switches, as depicted in Figures 9 and 15c, may be employed to conform a circuit that enables the control of an electronic device, such as a tablet, in order to perform determined functions. This system may be used in different applications, such as in infant learning stimulation, and in monitoring determined parameters like temperature, presence, sleeping conditions, etc.

The exemplary embodiment of the infant learning stimulation garment consists of at least one switch (1 P1 T, 1 P2T, or 1 P3T), in addition to other elements that enable the communication with the electronic device, or tablet, in a detachable or modular configuration, attachable by using the connectors or terminals within the garment wherein the TMB resides.

One of the 1 P1 T switches may connect to one of the 1 P2T switches, and both of these, in turn, connect to the terminals that will be in contact with the textile signal communications element, or module. If desired, one 1 P3T switch me be also utilized.

The connection between the different switches enables, by using combinational logic, the determination of the switch that the user activated, and therefore the transmission of a specific signal to the communications module, and by means of a computer program the control of the electronic device based on the received signal.

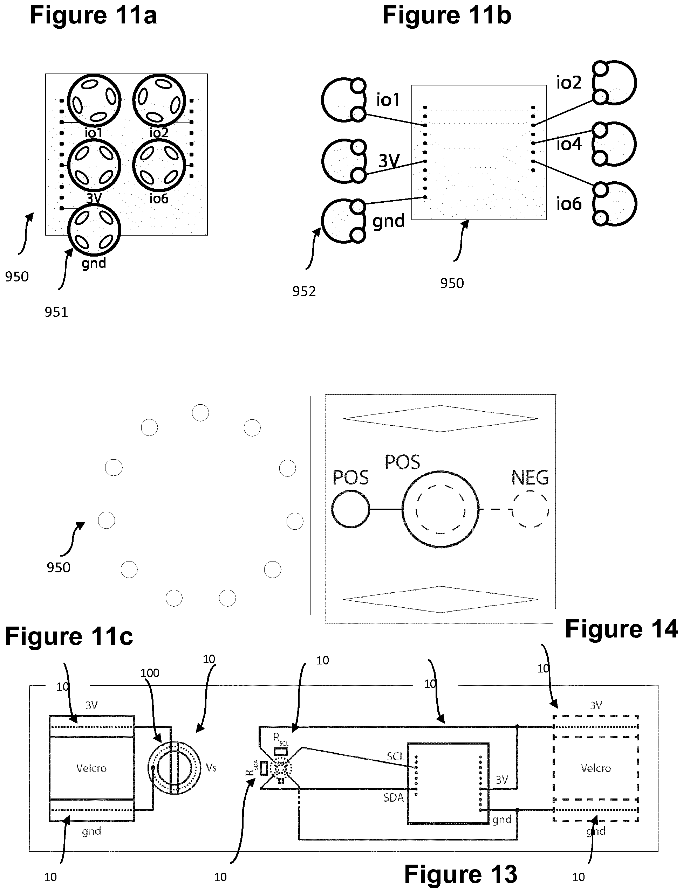

The arrangement of the switches in one of the layers of a TMB is better depicted in the exemplary embodiments of Figures 9 and 15c: the throw of one 1P1T (700) switch may be connected to the throw of one 1P2T (800) switch, and the throws of the latter switch may be connected to the throws of a 1 P3T (900) switch, in this manner at least three 1P1T (700) switches, three 1P2T (800) switches, and one 1P3T (900) switch may be utilized, thus enabling a TMB with 3 inputs to control up to 7 switches, achieving this performance due to the combinational logic, since the signals may be readily determined because the switches function as logic gates. The number of switches that can be implemented by means of the combinational logic is given by (2^*N -* 1), wherein N is the number of inputs available in the TMB design. In the previous example *N* = 3, thus giving a total of 7 available switches. The logical gates thus conformed may be associated with a specific signal value, for instance a binary series may be employed, as indicated in the figures (001, 010, 100), thus codifying and clearly determining the switch that was activated by the user. The TMB design may also incorporate connection buses 960 and interconnection sockets to accommodate the intercommunications modules, or control modules for a specific controller, the buses connections may be implemented by using fasteners or snaps or components normally employed in the textile industry, which may enable the communication of signals between the electric or electronic components of the TMB, or a TMB module; the sockets 950 may be like the ones illustrated in Figures 11a-11c. Conventional textile snaps or fasteners 951, 952 may be utilized in the interconnection of buses and optionally pads within a TMB.

The configuration of switches and conductive routings, or buses, that must be utilized to conform a specific arrangement, should become evident to those skilled in the art, in order to determine other optimal arrangements starting from this description.

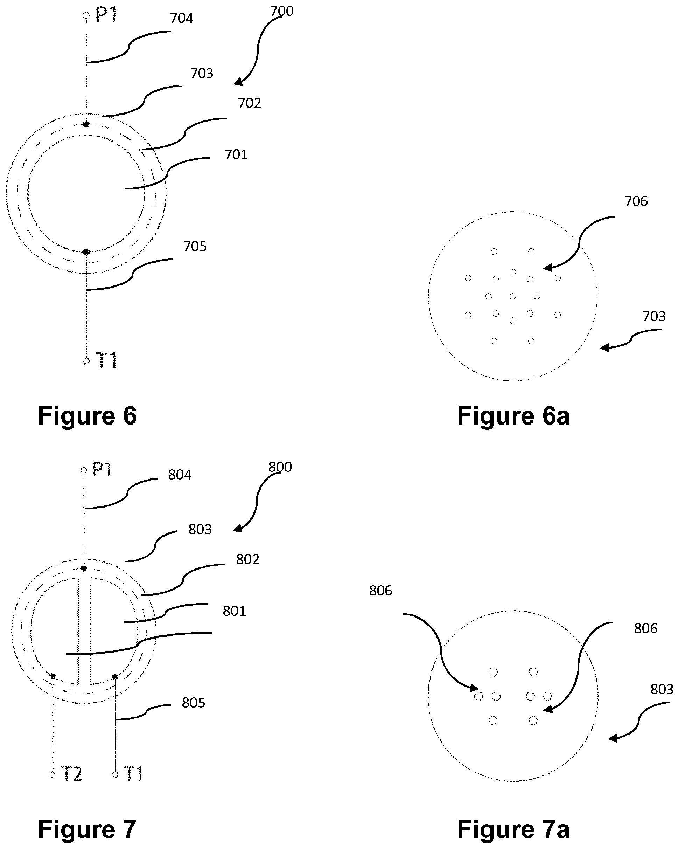

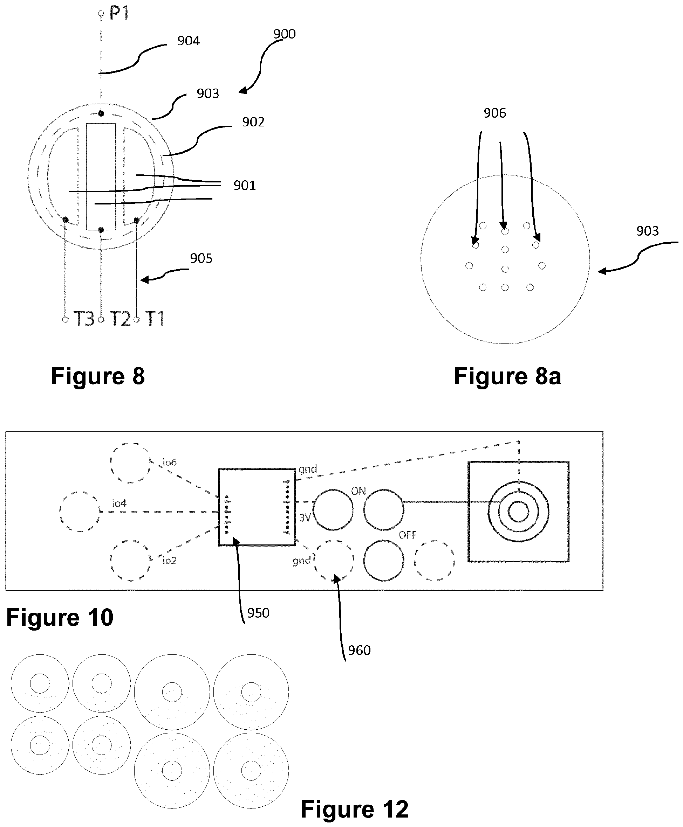

As depicted in Figures 6 and 8, the exemplary switches disclosed in the present invention are configured by a front or top layer (701, 801, 901), and a back or bottom layer (702, 802, 902), both layers, made of conductive textile material, are superposed and separated by a layer with isolating material (703, 803, 903), which may likewise be textile, such isolating material is configured to enable the communication between the two conductive layers (upper and lower), which is achieved by incorporating perforations (706, 806, 906) following a specific pattern, thus only enabling contact between certain regions of the upper and lower conductive layers.

The isolating materials (703, 803, 903) are designed with specific perforation patterns (706, 806, 906) in order to permit contact only in determined regions of the switch, as illustrated in the exemplary embodiments of Figures 6a, 7a, and 8a. In this fashion, in one 1P1T switch, the perforations 706 may be placed in a circular pattern, which enables contact in most of the conductive regions. In another 1 P2T switch, the pattern may be divided in order to generate two separate regions 806. Similarly, in one 1 P3T switch, the isolating material may be configured to exhibit three perforation regions 906.

Even though the switches are shown to have a circular form, the switches may adopt any form that is deemed necessary, and with the number of divisions needed to achieve the poles or throws required, taking into consideration that in both conductive layers, one, either the upper or lower, encompasses the complete area of the switch (indicated with dashed lines) (702, 802, 902) and the other, upper or lower, may encompass as many divisions as throws are required (indicated with solid lines) (701, 801, 901) Therefore in a 1P1T switch both layers are equal, in a 1 P2T one of the layers may have two sections 801, thus forming the necessary throws. In a 1 P3T, one of the layers may have three sections 901. These features may be seen in Figures 6 to 8. Evidently, both layers may be equally divided to generate switches with N poles and N throws, in order to conform an NPNT switch. Similarly, the isolating layer may have the necessary regions, perforations, and number according to the required contact regions of each switch.

The exemplary embodiment of the present disclosure shown in Figure 10 illustrates a communications module with an intercommunications socket 950 with communication nodes or connectors or buses 960.

The exemplary embodiment of the present disclosure illustrated in Figure 12 depicts contact zones or pads conformed of textile material, the pads are employed to join components, such as snaps, pins, or similar components, with the routings or buses of the TMB.

According to the exemplary embodiment of the present disclosure shown in Figure 13, a 2P2T switch may be implemented with the use of a hook and loop coupling element, such as Velcro®, conformed of conductive textile material, and functioning as a textile adhesive material, wherein one region functions as throw and the other as pole. This embodiment of the TMB may be utilized by a user as a bracelet, and the type of switch would correspond to one that is active when the hook and loop sections are joined, thus closing the circuit. The TMB depicted in Figure 13 is configured as a temperature sensing device, which monitors a user by locating the bracelet in a part of the user's body, the bracelet may, in turn, incorporate a module to communicate with a mobile device, such as a telephone or a tablet.

According to the exemplary embodiments of the present disclosure illustrated in Figures 15a through 15d, a TMB structure is shown, which may monitor bedroom textiles or linens, such as bedcovers or bed mattresses, which incorporate a TMB with an arrangement of 7 switches (700, 800, 900), which may be configured as previously described or in an alternating manner in order to monitor the sought after parameters, such as humidity presence. The isolating layer may incorporate perforation patterns 1501 to enable the electric communication between switches and contact pads 1502, 1503.

The exemplary embodiment of the present disclosure shown in Figure 9 may be configured as a system for infant learning stimulation, which by incorporating at least one switch, preferably 7 switches, as depicted, enables the infant, by using a garment suited to fit his size, to interact with an electronic device, such as a tablet, in order to wirelessly perform determined interactive tasks by using the device and the controls of the textile garment, for instance following the sequence of a tale. In this manner, instructions required by the electronic device may be commanded by using the TMB within the infant's garment, therefore enabling the infant to interact with the device and the TMB in order to develop cognitive abilities.

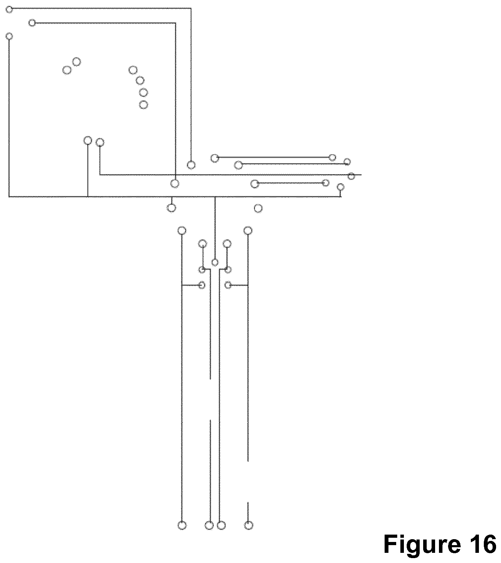

The exemplary embodiment of the present disclosure depicted in Figure 16 illustrates a TMB pattern in order to configure an infant monitor with humidity, movement, and temperature (textile) transducers, which may be incorporated to a garment, in order to determine if the infant has a fever, a change of diaper is necessary, or the infant is exhibiting voluntary or involuntary movement. In the same fashion, the embodiment may implement transducer modules to determine cardiac rhythm. The information may be communicated to a mobile device, such that the parents or persons caring for the infant may be able to monitor the infant activity. Appropriate sockets and buses are employed to perform the required monitoring.

The exemplary devices of the present disclosure may function with batteries and, therefore, a battery holder is depicted in Figure 14, wherein an emplacement for the batteries is provided, which is configured with a TMB that incorporates contact terminals or pads.

## Claims

1. A textile motherboard that consists of at least one substrate layer on which at least one peripheral is incorporated and one central processing unit, wherein the at least one peripheral is conformed of a textile switch, which consists of an isolating substrate with regions of null conductivity and regions of maximum conductivity.

2. Textile motherboard according to claim 1, wherein the textile motherboard incorporates a number of switches in a predetermined arrangement, which follows a pattern of logical gates.

3. Textile motherboard according to claim 2, wherein both the number and interconnections of textile switches are based on combinational logic.

4. Textile motherboard according to claim 3, wherein the number of switches is selected by employing the expression 2^*N* - 1, where N is the number of available inputs.

5. Textile motherboard according to claim 4, wherein the regions of maximum conductivity of the textile switch are arranged following specific patterns, which correspond to the perforations of the isolating substrate.

6. Textile motherboard according to claim 4, wherein the regions of minimum conductivity of the textile switch are arranged following specific patterns.

7. A system to stimulate learning that consists of a textile motherboard according to any of the claims 1 to 6, a textile intercommunications module, and an electronic device configured to receive signals coming from the textile motherboard, when the latter is operated by a user.

8. System according to claim 7, wherein the textile motherboard is incorporated into a garment and the switches enable the command of the device.

9. A system to monitor that consists of a textile motherboard according to any of the claims 1 to 6, wherein the substrate is configured as a garment or a bedroom textile or a bed linen.

10. A textile switch that consists of at least one conductive textile substrate material and an isolating substrate material, wherein the isolating material incorporates patterns to enable regions of maximum conductivity and regions of null conductivity.

11. Textile switch according to claim 10, wherein the conductive substrate and the regions of maximum conductivity of the isolating substrate coincide in number.

12. Textile switch according to claim 11, wherein the textile switch incorporates an additional conductive substrate.

13. Textile switch according to claim 12, wherein the isolating substrate is placed between the conductive substrates.

14. Textile switch according to claim 13, wherein the first substrate incorporates more than one conductive region.
